# EUROPÄISCHE PATENTSCHRIFT

(11) **EP 1 365 691 B1**
(45) Veröffentlichungstag und Bekanntmachung des Hinweises auf die Patenterteilung: **20.10.2004**
(21) Anmeldenummer: 03706471.4
(22) Anmeldetag: 11.02.2003
(51) Int. Cl.: A61B 17/32

(54) **MEDIZINISCHES INSTRUMENT**
MEDICAL INSTRUMENT
INSTRUMENT MEDICAL

(30) Priorität: 21.02.2002 DE 10207207
(43) Veröffentlichungstag der Anmeldung: 03.12.2003
(73) Patentinhaber: Karl Storz GmbH & Co. KG, 78532 Tuttlingen (DE)
(72) Erfinder: LANG, Dieter, 96342 Stockheim (DE); HOPF, Thomas, 96342 Stockheim (DE)
(74) Vertreter: Hofmeister, Frank Horst
(86) Internationale Anmeldenummer: PCT/EP2003/001303
(87) Internationale Veröffentlichungsnummer: WO 2003/070115

(56) Entgegenhaltungen:
- DE-A- 19 713 067
- DE-A- 19 915 427
- US-A- 4 712 545
- US-A- 4 994 024
- US-A- 5 613 977

## Beschreibung

Die Erfindung betrifft ein medizinisches Instrument mit einem hohlen Schaft, an dessen proximalem Ende eine aus mindestens zwei Griffteilen bestehende Handhabe angeordnet ist und an dessen distalem Ende ein aus mindestens zwei Maulteilen bestehendes Werkzeug angeordnet ist, wobei mindestens ein Maulteil des Werkzeugs zum Öffnen und Schließen über jeweils ein verschwenkbar ausgebildetes Griffteil der Handhabe gegenüber dem mindestens einen anderen Maulteil des Werkzeugs verschwenkbar ist und das mindestens eine verschwenkbare Maulteil und das zum Verschwenken des mindestens einen Maulteils dienende Griffteil der Handhabe über eine in dem hohlen Schaft gelagerte Zug-/ Schubstange miteinander verbunden sind und die Zug-/ Schubstange über das zugehörige mindestens eine verschwenkbare Griffteil der Handhabe ausschließlich in axialer Richtung verlagerbar ist.

Gattungsgemäße Instrumente lenken das bewegliche Maulteil bzw. die beweglichen Maulteile ausgehend von der Zug-/ Schubstange über einen Hebelmechanismus an. Der Winkel zwischen der Instrumentenachse und der Linie durch den Drehpunkt des Maulteils und des Angriffspunkts am Hebel wird dabei möglichst groß gewählt. Beim Öffnen oder Schließen der Maulteile kommt es aber zwangsläufig zu einer Verkleinerung des Winkels, was die Kraftübertragung deutlich verschlechtert. Aufgrund der Anlenkung der Zug-/ Schubstange am verstellbaren Griffteil der Handhabe, bei der das proximale Ende der Zug-/ Schubstange auf einer kreisbogenabschnittförmigen Bahn geführt wird, benötigt die Zug-/ Schubstange ein Höhenspiel innerhalb des hohlen Schaftes. Dieses Höhenspiel birgt aber die Gefahr des Verkantens und/oder Verdrehens der Zug-/ Schubstange innerhalb des hohlen Schaftes, wodurch das direkte Schnittgefühl des Operateurs deutlich beeinträchtigt werden kann.

Ein gattungsgemäßes medizinisches Instrument ist aus der US 5 490 861 A bekannt. Bei diesem medizinischen Instrument erfolgt der axiale Antrieb der Zug-/Schubstange mittels eines einer einerseits am verschwenkbaren Griffteil und andererseits am proximalen Ende der Zug-/Schubstange ausgebildeten Verzahnung. Beim Betätigen des verschwenkbaren Griffteil kämmt dessen Verzahnung mit der entsprechenden Verzahnung der Zug-/Schubstange und verlagert diese somit ausschließlich in axialer Richtung.

Neben dem rein axialen Antrieb der Zug-/Schubstange ist jedoch die verdrehsichere Lagerung der Zug-/Schubstange, durch die diese gegen Torsion geschützt werden soll, ausschlaggebend für eine einwandfreie Kraftübertragung auf das Werkzeug und ein gutes und sicheres Schnittgefühl für den Operateur. Diese erforderliche Torsionssteifigkeit ist bei dem aus US 5 490 861 A bekannten medizinischen Instrument nicht gegeben, da die zylindrische Zug-/Schubstange in einem Schaft mit zylindrischen Führungsquerschnitt für die Zug-/Schubstange gelagert ist, weshalb sich die Zug-/Schubstange beliebig innerhalb des Schaftes verdrehen kann. Auch die Wendelfeder am distalen Ende der Zug-/Schubstange gewährleistet nicht die gewünschte Verdrehsicherheit, vielmehr schwächt diese zudem nur aufwendig zu montierende Feder das Schnittgefühl für den Operateur, da die Feder ein Teil der Torsionskraft aufnimmt und es so dem Operateur unmöglich macht, die Schnittkraft des Werkzeugs mittels der Betätigung des Griffteils zu dosieren

Ein weiteres medizinisches Instrument ist aus der US 4,712,545 A bekannt. Bei diesem bekannten chirurgischen Instrument ist das kugelförmig verdickt ausgebildete proximale Ende der Zug-/ Schubstange im beweglichen Griffteil der Handhabe so gelagert, daß das proximale Ende der Zug-/ Schubstange auf einem Kreisbogenabschnitt um die Drehachse des beweglichen Griffteils bewegt wird. Aufgrund dieser Bahnführung und dem dadurch notwendigen Höhenspiel kann es leicht zu einem Verkanten der Zug-/ Schubstange innerhalb des Schaftes kommen. Darüber hinaus ist das bewegliche Maulteil im starren Teil des Schaftes gelagert, indem es auf einer Bogenbahn als Gleitlager gleitet. Diese Ausgestaltung führt zu gedachten Drehpunkten, die teilweise außerhalb des Instrumentenschafts liegen.

Durch diese Maßnahme wird allerdings nur der Hebelarm vergrößert - ähnliches kann auch durch Maßnahmen an der Handhabe realisiert werden - und damit in jeder Position mehr Kraft übertragen. Je kleiner allerdings der Winkel zwischen der Verbindungslinie Maulteildrehpunkt - Zug-/Schubstangenkraftangriffspunkt und der Instrumentenachse wird, umso geringer ist die Maulteilschließkraft.

Ausgehend von diesem Stand der Technik liegt der Erfindung die **Aufgabe** zugrunde, bei einfachem Aufbau des medizinischen Instruments die Zug/Schubstange zumindest abschnittweise verdrehsicher in dem hohlen Schaft zu lagern, um eine bestmögliche Kraftübertragung bei einem guten Schnittgefühl für den Operateur zu gewährleisten.

Die **Lösung** dieser Aufgabenstellung ist erfindungsgemäß dadurch gekennzeichnet, daß die Zug-/ Schubstange zumindest teilweise formschlüssig in eine formstabile Ummantelung eingesetzt ist, die ihrerseits zumindest abschnittsweise formschlüssig in den hohlen Schaft eingesetzt ist, und die Zug-/ Schubstange zumindest abschnittsweise verdrehsicher in der Ummantelung gelagert ist.

Durch das Einsetzen einer Ummantelung zur Aufnahme der Zug-/ Schubstange in den hohlen Schaft ist es auf konstruktiv einfache Art und Weise möglich, die Zug-/Schubstange verdrehsicher innerhalb des Schaftes anzuordnen, da es keiner konstruktiven Veränderungen des Schaftes bedarf, sondern lediglich die in den hohlen Schaft einzusetzende Ummantelung so auszugestalten ist, dass diese, beispielsweise durch eine an die Zug-/ Schubstange angepasste Formgebung der Querschnittsform eine Torsion der Zug-/ Schubstange verhindert. Vorteilhafterweise ist die Zug-/ Schubstange zudem zumindest abschnittsweise verdrehsicher in der Ummantelung gelagert

Darüber hinaus ermöglicht diese Ausgestaltungsform auch ein nachträgliches Ausrüsten eines im Durchmesser größeren hohlen Schaftes derart, daß eine spielfreie und verdrehsichere Lagerung der Zug-/ Schubstange gewährleistet ist.

Aufgrund der rein axialen Verlagerung der Zug-/ Schubstange ist es möglich, das medizinischen Instrument so auszugestalten, daß die Zug-/ Schubstange spielfrei in dem hohlen Schaft geführt ist.

Gemäß einer praktischen Ausführungsform der Erfindung wird hierzu vorgeschlagen, daß die Ummantelung beispielsweise eine im Querschnitt rechteckige Ausnehmung zur Aufnahme der im Querschnitt rechteckigen Zug-/ Schubstange aufweist.

Zur Ausbildung dieser rein axialen Verlagerung der Zug-/ Schubstange in distaler Richtung ist am verschwenkbaren Griffteil eine Druckfläche zur Kontaktierung einer Anlagefläche der Zug-/ Schubstange so ausgebildet, daß das verschwenkbare Griffteil die Zug-/ Schubstange exakt geradlinig in die distale Richtung drückt. Zum Verlagern der Zug-/ Schubstange in proximaler Richtung ist am verschwenkbaren Griffteil ein Mitnahmeelement angeordnet, das in eine in der Zug-/ Schubstange ausgebildete Ausnehmung eingreift und so die Zug-/ Schubstange ohne vertikale oder seitliche Auslenkung rein geradlinig wieder in proximaler Richtung zurückzieht.

Schließlich wird mit der Erfindung vorgeschlagen, daß der Durchmesser der Ummantelung zumindest der maximalen Höhe der Zug-/ Schubstange entspricht, so daß die Zug-/ Schubstange zur Verbesserung der Reinigbarkeit und zur Montageund Reparaturerleichterung nach dem Lösen des Werkzeugs zusammen mit der Ummantelung über das proximale Ende aus dem hohlen Schaft herausziehbar ist.

Weitere Merkmale und Vorteile der Erfindung ergeben sich aus der nachfolgenden Beschreibung der zugehörigen Zeichnung, in der ein Ausführungsbeispiel eines erfindungsgemäßen medizinischen Instruments nur beispielhaft schematisch dargestellt ist. In der Zeichnung zeigt:
- Fig. 1: eine Seitenansicht eines erfindungsgemäßen medizinischen Instruments, die Maulteile im geschlossenen Zustand darstellend;
- Fig. 2a: einen vergrößerten ausschnittweisen Längsschnitt durch das medizinische Instrument gemäß Fig. 1;
- Fig. 2b: eine Darstellung gemäß Fig. 2a, jedoch die Maulteile im geöffneten Zustand darstellend;
- Fig. 3a: eine Explosionsdarstellung des distalen Endes des medizinischen Instruments gemäß Fig. 1 und
- Fig. 3b: eine Explosionsdarstellung des proximalen Endes des medizinischen Instruments gemäß Fig. 1.

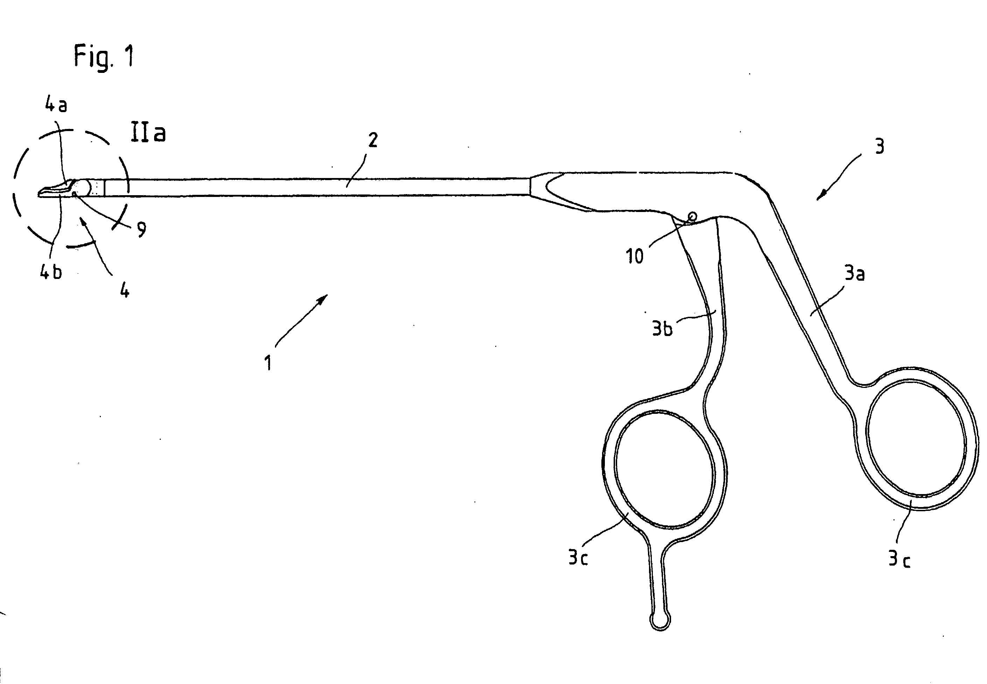

Die Abbildung Fig. 1 zeigt eine Seitenansicht eines medizinischen Instruments 1, dessen Kraftübertragungsmechanismus vielseitig verwendet werden kann, wie beispielsweise für Stanzen, Scheren, Nadelhalter, Faßinstrumente und dergleichen.

Das medizinische Instrument 1 besteht im wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, welches beim dargestellten Ausführungsbeispiel ein verschwenkbares Maulteil 4a und ein starr mit dem Schaft 2 verbundenes Maulteil 4b aufweist.

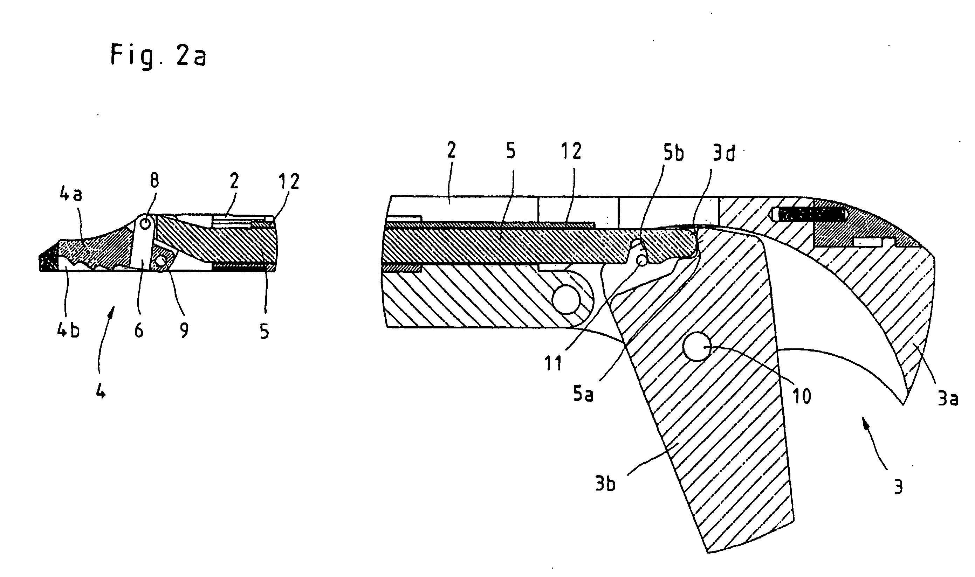

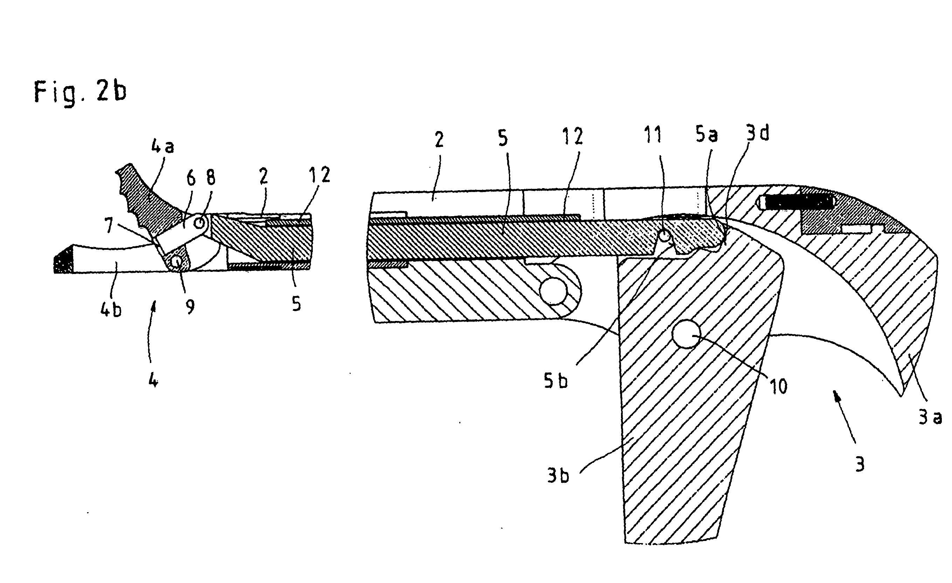

Wie aus den Detailansichten gemäß Fig. 2a und 2b ersichtlich, sind das verschwenkbare Maulteil 4a des Werkzeugs 4 und das verschwenkbare Griffteil 3b der Handhabe 3 über eine in dem hohlen Schaft 2 gelagerte Zug- / Schubstange 5 so miteinander verbunden, daß durch das Verschwenken des Griffteils 3b das verschwenkbare Maulteil 4a von der geschlossenen Stellung (Fig. 1 und 2a) in die offene Stellung (Fig. 2b) bzw. umgekehrt überführt werden kann.

Um eine möglichst gute Kraftübertragung zwischen der Handhabe 3 und dem verschwenkbaren Maulteil 4a des Werkzeugs 4 zu erzielen, sind, wie insbesondere aus Fig. 2b ersichtlich, die Zug-/ Schubstange 5 und das verschwenkbare Maultei 4a über einen Ausgleichshebel 6 miteinander verbunden, wobei der Ausgaeichshebei 6 verschiebbar in einer im verschwenkbaren Maulteil 4a ausgebildeten Führungsnut 7 gelagert ist und ein Verbindungspunkt 8 zwischen dem Ausgleichshebel 6 und der Zug-/ Schubstange 5 oberhalb eines Drehpunkts 9 des verschwenkbaren Maulteils 4a angeordnet ist. Selbstverständlich ist es auch möglich, das medizinische Instrument so auszugestalten, daß die Zug-/ Schubstange 5 direkt an dem verschwenkbaren Maulteil 4a angreift.

Die Verbindung von Zug-/ Schubstange 5 und verschwenkbarem Maulteil 4a über den verschiebbar im Maulteil 4a geführten Ausgleichshebel 6 ermöglicht ein gleichbleibendes Hebelverhältnis zwischen den beiden Bauteilen 5 und 4a, ohne die Gefahr einer Pendelbewegung der Zug-/ Schubstange 5. Aufgrund dieses gleichbleibenden Hebelverhältnisses hat der Operateur immer ein gutes und gleichmäßiges Schnittgefühl.

Zum sicheren Ergreifen der Griffteile 3a, 3b der Handhabe 3 weisen diese an ihren freien Enden Fingerösen 3c auf. Beim dargestellten Ausführungsbeispiel ist das Griffteil 3b um eine Schwenkachse 10 gegenüber dem anderen, starren Griffteil 3a verschwenkbar. Der Verschwenkweg der beiden Griffteile 3a, 3b zueinander läßt sich durch eine nicht dargestellte Übersetzung verkürzen. Ebenso ist es möglich, beide Griffteile 3a, 3b der Handhabe 3 als verschwenkbare Griffteile auszubilden. Durch die Kopplung des verschwenkbaren Griffteils 3b über die Zug-/ Schubstange 5 und den Ausgleichshebel 6 mit dem verschwenkbaren Maulteil 4a läßt sich das Werkzeug 4 beim Betätigen der Handhabe 3 öffnen und schließen.

Der Antrieb der Zug-/ Schubstange 5 erfolgt derart, daß diese zum Betätigen des verschwenkbaren Maulteils 4a rein axial innerhalb des hohlen Schafts 2 verlagerbar ist. Wie aus dem Vergleich der Abbildungen Fig. 2a und 2b ersichtlich, wird die Zug-/ Schubstange 5 zum Schließen des verschwenkbaren Maulteils 4a über das verschwenkbare Griffteil 3b in die distale Richtung geschoben. Hierzu ist an dem verschwenkbaren Griffteil 3b eine Druckfläche 3d ausgebildet, die mit einer entsprechenden Anlagefläche 5a an der Zug-/ Schubstange 5 zusammenwirkt. Im Gegensatz zum Stand der Technik, bei dem das proximale Ende der Zug-/ Schubstange 5 einen kugelförmigen Lagerkopf aufweist, der in einer entsprechenden Lageraufnahme des verschwenkbaren Griffteils 3b gelagert ist, was zu einer Bewegung des proximalen Endes der Zug-/ Schubstange 5 auf einer kreisbogenabschnittförmigen Bahn führt, ist es durch die Ausbildung der Druckfläche 3d am verschwenkbaren Griffteil 3b einerseits und der Anlagefläche 5a an der Zug-/ Schubstange 5 andererseits möglich, die Bewegung der Zug-/ Schubstange 5 auf eine rein axiale Bewegung zu reduzieren.

Das Zurückziehen der Zug-/ Schubstange 5 in proximaler Richtung zum Überführen des verschwenkbaren Maulteils 4a von der in Fig. 2a dargestellten geschlossenen Stellung in die geöffnete Stellung gemäß Fig. 2b erfolgt über ein am verschwenkbaren Griffteil 3b angeordnetes Mitnahmeelement 11, das in eine in der Zug-/ Schubstange 5 ausgebildete Ausnehmung 5b eingreift. Diese reine Zugbewegung bewirkt ebenfalls eine Bewegung der Zug-/ Schubstange 5 ausschließlich in axialer Richtung.

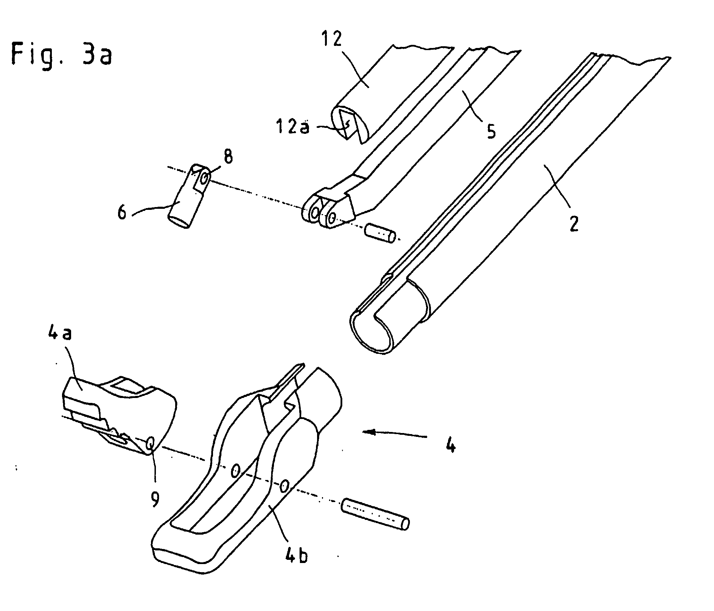

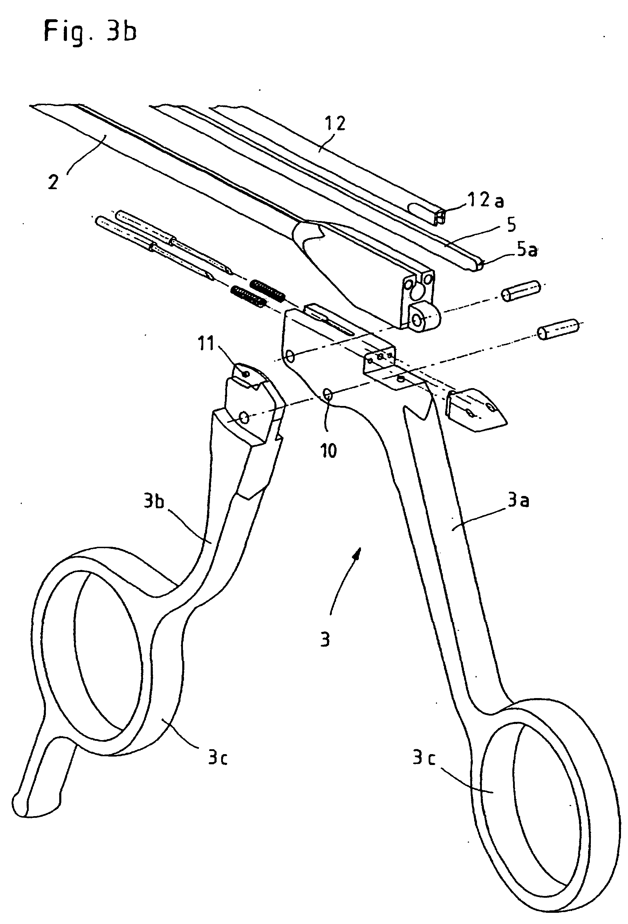

Da aufgrund der rein axialen Führung der Zug-/ Schubstange 5 keine Pendelbewegungen der Zug-/ Schubstange 5 auftreten, besteht die Möglichkeit, die Zug-/Schubstange 5 spielfrei in dem hohlen Schaft 2 zu lagern. Hierzu ist eine Ummantelung 12 vorgesehen, in die die Zug-/ Schubstange 5 zumindest teilweise formschlüssig eingesetzt ist, wobei die Ummantelung 12, ihrerseits zumindest abschnittsweise formschlüssig in den hohlen Schaft 2 eingesetzt ist, wie dies den Explosionsdarstellungen gemäß Fig. 3a und 3b zu entnehmen ist.

Um weiterhin sicherzustellen, daß die Zug-/ Schubstange 5 auch torsionsfrei, das heißt verdrehsicher innerhalb des Schafts 2 führbar ist, ist bei der dargestellten Ausführungsform eine Ausnehmung 12a zur Aufnahme der Zug-/ Schubstange 5 in der Ummantelung 12 so ausgebildet, daß die Innenkontur der Ausnehmung 12a rechteckig ausgebildet, der Außenkontur der ebenfalls rechteckigen Zug-/ Schubstange 5 entspricht. Die Verwendung der Ummantelung 12 bietet die Möglichkeit, die spielfreie Führung der Zug-/ Schubstange 5 innerhalb des Schaftes 2 auch dann sicherzustellen, wenn der Innendurchmesser des hohlen Schaftes 2 vom Außendurchmesser der Zug-/ Schubstange 5 abweicht. Überdies ist die Herstellbarkeit der einzelnen Bauteile bei einer Ausgestaltung der Zug-/Schubstange 5 mit Rechteckquerschnitt deutlich erleichtert.

Das Reinigen und Reparieren des medizinischen Instruments 1 wird dadurch erleichtert, daß die Zug-/ Schubstange 5 und der Ausgleichshebel 6 als komplette Einheit aus dem hohlen Schaft 2 entnehmbar sind. Zu diesem Zweck ist es vorteilhaft, wenn der Durchmesser der Ummantelung 12 zumindest der maximalen Höhe der Zug-/ Schubstange 5 entspricht, so daß die Zug-/ Schubstange 5 nach dem Lösen des Werkzeugs 4 aus dem proximalen Ende des Schaftes 2 herausziehbar ist.

Insgesamt zeichnet sich das dargestellte medizinische Instrument 1 dadurch aus, daß es eine gleichmäßig starke Kraftübertragung unabhängig von der Öffnungsstellung der Maulteile gewährleistet und es möglich ist, diese Kraft dosiert einzusetzen, so daß nach dem Durchtrennen harten Gewebes kein unkontrolliertes Durchschlagen der kraftbeanspruchten Maulteile erfolgt.

### Bezugszeichenliste

- 1: medizinisches Instrument
- 2: Schaft
- 3: Handhabe
- 3a: starres Griffteil
- 3b: verschwenkbares Griffteil
- 3c: Fingeröse
- 3d: Druckfläche
- 4: Werkzeug
- 4a: verschwenkbares Maulteil
- 4b: starres Maulteil
- 5: Zug- / Schubstange
- 5a: Anlagefläche
- 5b: Ausnehmung
- 6: Ausgleichshebel
- 7: Führungsnut
- 8: Verbindungspunkt
- 9: Drehpunkt
- 10: Schwenkachse
- 11: Mitnahmeelement
- 12: Ummantelung
- 12a: Ausnehmung

## Patentansprüche

1. Medizinisches Instrument mit einem hohlen Schaft (2), an dessen proximalem Ende eine aus mindestens zwei Griffteilen (3a, 3b) bestehende Handhabe (3) angeordnet ist und an dessen distalem Ende ein aus mindestens zwei Maulteilen (4a, 4b) bestehendes Werkzeug (4) angeordnet ist, wobei mindestens ein Maulteil (4a) des Werkzeugs (4) zum Öffnen und Schließen über jeweils ein verschwenkbar ausgebildetes Griffteil (3b) der Handhabe (3) gegenüber dem mindestens einen anderen Maulteil (4b) des Werkzeugs (4) verschwenkbar ist und das mindestens eine verschwenkbare Maulteil (4a) und das zum Verschwenken des mindestens einen Maulteils (4a) dienende Griffteil (3b) der Handhabe (3) über eine in dem hohlen Schaft (2) gelagerte Zug-/Schubstange (5) miteinander verbunden sind und die Zug-/ Schubstange (5) über das zugehörige mindestens eine verschwenkbare Griffteil (3b) der Handhabe (3) ausschließlich in axialer Richtung verlagerbar ist,
**dadurch gekennzeichnet,**
**daß** die Zug-/ Schubstange (5) zumindest teilweise formschlüssig in eine formstabile Ummantelung (12) eingesetzt ist, die ihrerseits zumindest abschnittsweise formschlüssig in den hohlen Schaft (2) eingesetzt ist, und die Zug-/ Schubstange (5) zumindest abschnittsweise verdrehsicher in der Ummantelung (12) gelagert ist.

2. Medizinisches Instrument nach Anspruch 1, **dadurch gekennzeichnet, daß** die Ummantelung (12) eine im Querschnitt rechteckige Ausnehmung (12a) zur Aufnahme der im Querschnitt rechteckigen Zug-/ Schubstange (5) aufweist.

3. Medizinisches Instrument nach Anspruch 1 oder 2, **dadurch gekennzeichnet, daß** der Durchmesser der Ummantelung (12) zumindest der maximalen Höhe der Zug-/ Schubstange (5) entspricht.

4. Medizinisches Instrument nach einem der Ansprüche 1 bis 3, **dadurch gekennzeichnet, daß** zum Verlagern der Zug-/ Schubstange (5) in distaler Richtung am verschwenkbaren Griffteil (3b) eine Druckfläche (3d) zur Kontaktierung einer Anlagefläche (5a) der Zug-/ Schubstange (5) ausgebildet ist.

5. Medizinisches Instrument nach Anspruch 4, **dadurch gekennzeichnet, daß** zum Verlagern der Zug-/ Schubstange (5) in proximaler Richtung am verschwenkbaren Griffteil (3b) ein Mitnahmeelement (11) angeordnet ist, das in eine in der Zug-/ Schubstange (5) ausgebildete Ausnehmung (5b) eingreift.

6. Medizinisches Instrument nach einem der Ansprüche 1 bis 5, **dadurch gekennzeichnet, daß** die Zug-/ Schubstange (5) als Einheit aus dem Schaft (2) entnehmbar ist.

## Claims

1. Medical instrument with a hollow shaft (2) at whose proximal end there is a handle (3) consisting of at least two grip parts (3a, 3b), and at whose distal end there is a tool (4) consisting of at least two jaw parts (4a, 4b), at least one jaw part (4a) of the tool (4) being pivotable relative to the at least one other jaw part (4b) of the tool (4) for the purpose of opening and closing via a respective pivotable grip part (3b) of the handle (3), and the at least one pivotable jaw part (4a) and the grip part (3b) of the handle (3) serving for pivoting the at least one jaw part (4a) being connected to one another via a pull/push rod (5) mounted in the hollow shaft (2), and the pull/push rod (5) being displaceable only in the axial direction via the associated at least one pivotable grip part (3b) of the handle (3), **characterized in that** the pull/push rod (5) is inserted at least in some parts with a form fit into a dimensionally stable jacket (12) which in turn is inserted, at least in some sections with a form fit, into the hollow shaft (2), and the pull/push rod (5) is mounted secure against turning at least in some sections in the jacket (12).

2. Medical instrument according to Claim 1, **characterized in that** the jacket (12) has a recess (12a) of rectangular cross section for receiving the pull/push rod (5) of rectangular cross section.

3. Medical instrument according to Claim 1 or 2, **characterized in that** the diameter of the jacket (12) corresponds at least to the maximum height of the pull/push rod (5).

4. Medical instrument according to one of Claims 1 to 3, **characterized in that**, in order to displace the pull/push rod (5) in the distal direction, a pressing surface (3d) is formed on the pivotable grip part (3b) for making contact with a bearing surface (5a) of the pull/push rod (5).

5. Medical instrument according to Claim 4, **characterized in that**, in order to displace the pull/push rod (5) in the proximal direction, a carrier element (11) is arranged on the pivotable grip part (3b) and engages in a recess (5b) formed in the pull/push rod (5).

6. Medical instrument according to one of Claims 1 to 5, **characterized in that** the pull/push rod (5) can be removed as one unit from the shaft (2).

## Revendications

1. Instrument médical comportant un tube, sur l'extrémité proximale duquel est disposée une poignée (3) formée par au moins deux parties de préhension (3a, 3b) et sur l'extrémité distale duquel est disposé un outil (4) constitué par au moins deux éléments de mâchoire (4a, 4b), dans lequel au moins un élément de mâchoire (4a) de l'outil (4) peut pivoter, pour l'ouverture et la fermeture au moyen respectivement d'une partie de préhension (3b) de la poignée (3) montée pivotante, par rapport à au moins un autre élément de mâchoire (4b) de l'outil (4), et le au moins un élément de mâchoire pivotant (4a) et la partie de préhension (3b) de la poignée (3), utilisée pour faire pivoter le au moins un élément de mâchoire (4a), sont reliées entre eux par l'intermédiaire d'une tige de traction/poussée montée dans le tube creux (2), et la tige de traction/poussée (5) est déplaçable exclusivement dans une direction axiale au moyen du au moins une partie de préhension pivotante associée (3b) de la poignée (3),
**caractérisé en ce que** la tige de traction/poussée (5) est insérée au moins en partie selon une liaison par formes complémentaires dans une enveloppe de forme de stable (12), qui pour sa part est insérée au moins par endroits selon une liaison par formes complémentaires dans le tube creux (2), et la tige de traction/poussée (5) est montée au moins par endroits de manière à être bloquée en rotation dans l'enveloppe (12).

2. Instrument médical selon la revendication 1, **caractérisé en ce que** l'enveloppe (12) possède un évidement (12a) rectangulaire en coupe transversale, servant à loger la tige de poussée/traction (5) rectangulaire en coupe transversale.

3. Instrument médical selon la revendication 1 ou 2, **caractérisé en ce que** le diamètre de l'enveloppe (12) correspond au moins à la hauteur maximale de la tige de traction/poussée (5).

4. Instrument médical selon l'une des revendications 1 à 3, **caractérisé en ce que** pour le déplacement de la tige de traction/poussée (5) dans une direction distale, une surface de pression (3d) destinée à venir en contact avec une surface d'application (5a) de la tige de traction/poussée (5) est formée sur la partie de préhension pivotante (3b).

5. Instrument médical selon la revendication 4, **caractérisé en ce que** pour le décalage de la tige de traction/poussée (5) dans la direction proximale, sur la partie de préhension pivotante (3b) est disposé un organe d'entraînement (11) qui s'engage dans un évidement (5b) formé dans la tige de traction/poussée (5).

6. Instrument médical selon l'une des revendications 1 à 5, **caractérisé en ce que** la tige de traction/poussée (5) peut être tirée du tube (2) sous la forme d'une unité.
